# FASCICULE DE BREVET EUROPEEN

(11) **EP 4 167 902 B1**
(45) Date de publication et mention de la délivrance du brevet: **14.08.2024**
(21) Numéro de dépôt: 21740122.3
(22) Date de dépôt: 17.06.2021
(51) Int. Cl.: A61F 2/00, A61F 5/48

(54) **DISPOSITIF MEDICAL ET METHODE DE DETECTION D'UN CHANGEMENT DE POSITION**
MEDIZINISCHE VORRICHTUNG UND VERFAHREN ZUR ERKENNUNG EINER POSITIONSÄNDERUNG
MEDICAL DEVICE AND METHOD OF DETECTING A CHANGE OF POSITION

(30) Priorité: 19.06.2020 FR 2006429
(43) Date de publication de la demande: 26.04.2023
(73) Titulaire: Uromems, 38320 Eybens (FR)
(72) Inventeur: LAMRAOUI, Hamid, 38410 Vaulnaveys le Haut (FR); ROUSTAN, Benjamin, 38330 Saint Nazaire Les Eymes (FR)
(74) Mandataire: Regimbeau
(86) Numéro de dépôt international: PCT/FR2021/051086
(87) Numéro de publication internationale: WO 2021/255388

(56) Documents cités:
- EP-A1- 1 584 303
- EP-A1- 3 223 748
- EP-B1- 3 223 748

## Description

### DOMAINE DE L'INVENTION

La présente invention concerne un dispositif médical implantable dans un corps humain ou animal.

L'invention concerne en particulier les dispositifs médicaux implantables actifs, c'est-à-dire les dispositifs médicaux dépendant pour leur fonctionnement d'une source d'énergie électrique et leur configuration pour la détection d'un changement de position d'au moins une partie du corps humain ou animal.

### ETAT DE LA TECHNIQUE

Il existe de nombreux dispositifs médicaux implantables pour remplir une fonction dans le corps du patient, par exemple pour pallier le dysfonctionnement d'un organe naturel comme celui d'un sphincter dans le cas de l'incontinence urinaire ou pour agir en appliquant une thérapie particulière comme c'est le cas d'un stimulateur cardiaque ou d'un neurostimulateur.

En particulier, le cas de l'incontinence urinaire est un handicap qui touche aussi bien les femmes que les hommes. Ce handicap peut être défini comme une perte involontaire d'urine par l'urètre. Dans la plupart des cas, cela est dû à un affaiblissement du support pelvien ou du bloc vessie/sphincter. Selon les symptômes, il existe des solutions ne nécessitant aucune intervention chirurgicale, telles que la rééducation ou des traitements médicamenteux. Lorsque ces méthodes ne suffisent pas, l'incontinence est dite « sévère » et nécessite alors la pose d'une prothèse pour permettre de rendre au patient une vie sociale normale. En cas d'incontinence sévère, la méthode la plus employée consiste à mettre en place un sphincter urinaire artificiel.

On connait des dispositifs médicaux implantables destinés à obturer sélectivement un conduit anatomique, par exemple pour remédier à une incontinence (cas des sphincters urinaires et anaux artificiels). L'obturation du conduit anatomique est généralement engendrée par la compression exercée par une manchette enroulée autour dudit conduit.

De nos jours, le sphincter urinaire artificiel le plus répandu consiste en un système hydraulique passif en élastomère de silicone composé de trois parties principales.

La première partie consiste en un élément déformable de type manchette occlusive placée autour de l'urètre, éventuellement du col vésical lors d'une implantation chez la femme, exerçant une pression circonférentielle urétrale grâce à un coussin rempli de liquide assurant ainsi la continence du patient. La deuxième est un ballon de régulation qui permet, lorsqu'il est rempli avec un certain volume de liquide, de créer une pression hydraulique constante. La pression de régulation est choisie en fonction du patient pendant l'opération, celle-ci ne pouvant plus être modifiée une fois la prothèse implantée. Il faut enfin une pompe manuelle, la troisième partie, assurant l'ouverture de la partie urétrale comprimée par la manchette. Cette pompe est composée d'une poire, d'un résistor et de deux valves qui assurent la circulation du liquide vers ou depuis la manchette occlusive. Lorsque le patient ressent le besoin d'uriner, il comprime la poire située sur la partie inférieure de la pompe, le fluide est transféré de la manchette vers le ballon de régulation : la pression exercée sur l'urètre devient alors négligeable devant la pression vésicale. L'urine peut alors s'écouler librement hors de la vessie. En pratique, quelques minutes après, le liquide est transféré du ballon vers la manchette grâce à la pression exercée sur le résistor par le ballon de régulation, l'urètre est de nouveau occlus.

Cette prothèse passive présente un certain nombre d'inconvénients.

D'abord, la pression de régulation de la manchette ne peut être adaptée qu'au moment de la pose de la prothèse, ce qui pourra poser des problèmes si la pathologie évolue dans le temps de sorte que la pression au niveau de l'urètre devrait être modifiée pour répondre aux besoins du patient ou même en cas de changement de posture ou d'activité physique pouvant entrainer une variation de la pression exercée sur le sphincter et sur la manchette.

En outre, ce système n'offre pas un confort satisfaisant car le patient doit actionner la pompe à chaque fois que cela est nécessaire, la commande n'étant de surcroit pas aisée puisqu'il convient de maintenir la pompe manuellement pendant la miction d'une part et de la presser avec force d'autre part. En effet, cela est d'autant plus problématique que la pompe de contrôle se trouve chez l'homme dans le scrotum et chez la femme dans l'une des grandes lèvres.

Enfin, le fonctionnement de cette prothèse passe par une compression importante de l'urètre de manière quasi continue, quels que soient la posture et le niveau de compression nécessaire, ce qui peut induire des atrophies urétrales. En effet, la prothèse ne fonctionnant qu'à une seule pression urétrale, celle-ci doit être assez importante pour éviter les fuites, ce qui à terme peut endommager l'urètre.

De plus, pour que la miction soit possible, le patient doit contracter suffisamment sa vessie pour lutter contre la résistance créée au niveau de l'urètre par la prothèse. Outre l'inconvénient mentionné plus haut de compression quasi continue de l'urètre quel que soit la posture, l'effort requis pour la miction avec ce type de prothèse crée une sorte d'adénome de prostate artificiel qui peut avoir des conséquences graves pour le patient.

Il existe enfin des problèmes de fiabilité des éléments constituant la prothèse elle-même, notamment de l'ensemble pompe et circuit hydraulique.

Ces inconvénients des sphincters urinaires artificiels existants impliquent que ces prothèses ne sont utilisées que pour traiter les incontinences urinaires importantes. En conséquence, les sphincters urinaires artificiels ne sont pas utilisés pour les patients souffrant d'une faible incontinence et ils ne sont pas adaptés à toutes les situations et postures. En effet ces derniers préfèrent subir la gêne occasionnée par leur pathologie.

Des dispositifs alternatifs actifs ont été développés pour tenter de remédier à certains de ces inconvénients, en proposant notamment des commandes électroniques de la prothèse.

Parmi ces différents types de dispositifs médicaux implantés certains incluent des capteurs de posture du corps humain ou animal de sorte à adapter la pression exercée sur le conduit naturel par la manchette occlusive. L'adaptation de la pression exercée s'avère indispensable pour remédier aux inconvénients décrits ci-dessus et pour gérer l'incontinence d'effort, c'est-à-dire lorsque le patient exerce une activité qui entraine une variation de pression sur son urètre et sur la manchette, ou pour réduire la pression lorsque le patient est en position allongé au repos de sorte à ne pas solliciter l'urètre inutilement ce qui pourrait engendrer, comme expliqué, une atrophie sur le long terme.

A cet effet, il devient nécessaire de détecter la position du patient et d'adapter la pression exercée faute de quoi les organes subiront une détérioration importante ou d'adapter la thérapie appliquée en fonction de la position du patient.

Par exemple, la détection de la position du corps peut être effectuée au moyen d'un accéléromètre prévu dans le dispositif médical. L'accéléromètre peut ainsi mesurer une accélération notamment gravitationnelle selon une ou de préférence trois directions permettant de discriminer différentes postures du corps du patient. Ensuite par comparaison avec des valeurs de référence et des calculs de variation de valeurs d'angle, on peut en déduire une position du corps. Toutefois l'utilisation des accéléromètres à ces fins conduit à des résultats qui peuvent montrer une fiabilité relative et des imprécisions importantes, en particulier dans le cas de migration ou de rotation du dispositif médical implanté. EP 1 584 303 A1 décrit un dispositif médical agencé pour être implanté dans un corps humain ou animal selon le préambule de la revendication 1.

### EXPOSE DE L'INVENTION

La présente invention a donc pour but de remédier aux inconvénients détaillés ci-dessus en proposant un dispositif médical actif implantable et une méthode de détection de la position qui soit plus fiable et plus robuste dans le temps.

A cet effet la présente invention propose un dispositif médical agencé pour être implanté dans un corps humain ou animal, comprenant un boiter, au moins un élément déformable, une connexion tubulaire assurant une liaison fluidique entre le boîtier et l'élément déformable pour former un circuit fluidique, et au moins un capteur apte à mesurer ou calculer une pression dans le circuit fluidique, le boîtier et l'élément déformable étant adaptés pour être implantés dans le corps humain ou animal distants l'un de l'autre, le dispositif médical étant caractérisé en ce qu'il est configuré pour détecter une position et/ou un changement de position d'au moins une partie dudit corps en fonction d'une variation de la pression mesurée ou calculée par le capteur.

De préférence, la détection d'une position et/ou d'un changement de position d'au moins une partie du corps correspond à la détection d'une posture et/ou d'un changement de posture dudit corps. Dans ce qui suit, on décrira donc principalement les postures et changements de posture.

Toutefois, il est entendu que ce qui suit peut également s'appliquer à un changement de position d'une partie du corps seulement. Par exemple, le dispositif selon l'invention est apte à détecter la position d'une prothèse d'une jambe mécatronique. Son mouvement peut donc être adapté en conséquence.

Selon un mode de réalisation de l'invention, le boîtier est rigide et lié de manière hermétique à une extrémité de ladite connexion tubulaire.

Selon un mode de réalisation de l'invention, l'élément déformable est gonflable.

Selon un mode de réalisation de l'invention, le capteur est disposé à l'intérieur du boîtier et apte à mesurer ou calculer la pression dans l'élément déformable.

Selon un mode de réalisation particulier de l'invention, le boîtier comprend un réservoir de fluide incluant une partie mobile et un actionneur de la partie mobile pour faire varier un volume dudit réservoir, le capteur apte à mesurer ou calculer la pression étant lié mécaniquement à ladite partie mobile et/ou à l'actionneur, notamment de manière solidaire.

Selon un mode de réalisation de l'invention, le boîtier et l'élément déformable sont adaptés pour être implantés dans le corps humain ou animal distants l'un de l'autre selon une direction qui varie lors d'un changement de position d'au moins une partie dudit corps, notamment lors du passage d'une position debout à allongée. De préférence, le boîtier et l'élément déformable sont adaptés pour être implantés dans le corps humain ou animal distants l'un de l'autre selon une direction sensiblement équivalente à une direction longitudinale dudit corps. Encore de préférence, le boîtier et l'élément déformable sont adaptés pour être implantés dans le corps humain ou animal distants l'un de l'autre d'une distance d'au moins 5 cm, de préférence d'une distance d'au moins 10 cm, encore de préférence d'une distance d'au moins de 20 cm.

Selon un mode de réalisation particulier de l'invention, le dispositif comprend un capteur de position d'au moins une partie dudit corps humain ou animal.

Selon un mode de réalisation particulier de l'invention, le dispositif comprend un capteur de position d'au moins une partie du corps humain ou animal, le dispositif étant configuré pour détecter la position et/ou le changement de position d'au moins une partie dudit corps à partir d'une mesure ou d'un calcul de pression par le capteur apte à mesurer ou calculer la pression et pour confirmer ladite position et/ou ledit changement de position à partir d'une mesure du capteur de position.

Selon un autre mode de réalisation particulier de l'invention, le dispositif comprend un capteur de position d'au moins une partie du corps humain ou animal, le dispositif étant configuré pour détecter la position et/ou le changement de position d'au moins une partie dudit corps par le capteur de position et pour confirmer ladite position et/ou ledit changement de position à partir d'une mesure ou d'un calcul de la pression dans le circuit fluidique par le capteur apte à mesurer ou calculer la pression.

Dans un mode de réalisation de l'invention, le capteur de position est choisi parmi : un accéléromètre, notamment un accéléromètre agencé pour calculer les accélérations linéaires selon trois axes orthogonaux, un gyroscope et/ou un inclinomètre.

Dans un mode de réalisation de l'invention, le dispositif comprend une unité de commande configurée pour réaliser au moins une des opérations suivantes :
- mesurer ou calculer une pression au moyen du capteur apte à mesurer ou calculer une pression,
- détecter une position ou un changement de position d'au moins une partie dudit corps en fonction d'un capteur de position et/ou du capteur apte à mesurer ou calculer la pression.

Dans un mode de réalisation de l'invention, l'unité de commande est configurée pour commander l'actionneur pour déplacer la partie mobile du réservoir et faire varier le volume dudit réservoir.

Selon un mode de réalisation particulier de l'invention, l'élément déformable est une manchette occlusive configurée pour obturer un conduit naturel dudit corps humain ou animal. De préférence, le dispositif est configuré pour être implanté dans un corps humain ou animal pour obturer un conduit naturel dudit corps humain ou animal compris parmi au moins : un urètre, un conduit gastrique, un colon ou un rectum.

Selon un mode de réalisation particulier de l'invention, le dispositif est configuré pour agir en tant que stimulateur cardiaque ou tant que neurostimulateur.

L'invention s'étend à une méthode de détection d'une position et/ou d'un changement de position d'au moins une partie d'un corps humain ou animal, dans laquelle ledit corps comprend un dispositif médical comprenant un boîtier, au moins un élément déformable, une connexion tubulaire assurant une liaison fluidique entre le boîtier et l'élément déformable pour former un circuit fluidique, et au moins un capteur apte à mesurer ou calculer une pression dans le circuit fluidique, le boîtier et l'élément déformable étant agencés à distance l'un de l'autre, dans laquelle on détecte une position et/ou un changement de position d'au moins une partie dudit corps en fonction d'une variation de la pression mesurée ou calculée par le capteur apte à mesurer ou calculer une pression.

Dans un mode de réalisation de la méthode selon l'invention, le boîtier et l'élément déformable dans le corps humain ou animal sont distants l'un de l'autre selon une direction qui varie lors d'un changement de position d'au moins une partie dudit corps, notamment lors du passage d'une position debout à allongée.

Dans un autre mode de réalisation de la méthode selon l'invention, le boîtier et l'élément déformable dans le corps humain ou animal sont distants l'un de l'autre selon une direction sensiblement équivalente à une direction longitudinale dudit corps.

Dans encore un autre mode de réalisation particulier de la méthode selon l'invention, équipe le dispositif médical d'une unité de commande configurée qui réalise au moins une des opérations suivantes :
- mesurer ou calculer une pression au moyen du capteur apte à mesurer ou calculer une pression,
- détecter une position et/ou un changement de position d'au moins une partie dudit corps en fonction d'un capteur de position et/ou du capteur apte à mesurer ou calculer la pression.

Dans un autre mode de réalisation de la méthode selon l'invention, l'unité de commande est configurée pour commander un actionneur pour déplacer une partie mobile d'un réservoir et faire varier le volume dudit réservoir.

Dans un autre mode de réalisation de la méthode selon l'invention, l'élément déformable est gonflable et choisi parmi une manchette occlusive agencée pour obturer un conduit naturel dudit corps humain ou animal, une prothèse pénienne ou un élément déformable configuré pour limiter une entrée d'aliments dans un estomac du corps humain ou animal.

Dans un autre mode de réalisation particulier de la méthode selon l'invention, on calibre un capteur de position apte à détecter une position et/ou un changement de position d'au moins une partie du corps au moyen du capteur apte à mesurer ou calculer une pression.

Enfin dans un autre mode de réalisation particulier de la méthode selon l'invention, l'élément déformable est gonflable et choisi parmi une manchette occlusive agencée pour obturer un conduit naturel dudit corps humain ou animal.

### DESCRIPTION DES FIGURES

D'autres caractéristiques et avantages de l'invention ressortiront de la description détaillée qui va suivre, en référence aux dessins annexés sur lesquels :
- la figure 1 illustre une vue schématique d'un dispositif médical du type sphincter artificiel selon un exemple de réalisation de l'invention ;
- la figure 2 illustre une vue schématique de la position du dispositif médical de la figure 1 implanté dans le corps humain vue de face ;
- la figure 3 illustre une vue détaillée du dispositif médical implanté dans le corps d'un homme en position debout vu de profil ;
- la figure 4 illustre une vue détaillée du dispositif médical implanté dans le corps d'un homme en position décubitus dorsal vu de profil ;
- la figure 5 illustre la méthode de détection selon l'invention.

### DESCRIPTION DETAILLEE DE MODES DE REALISATION

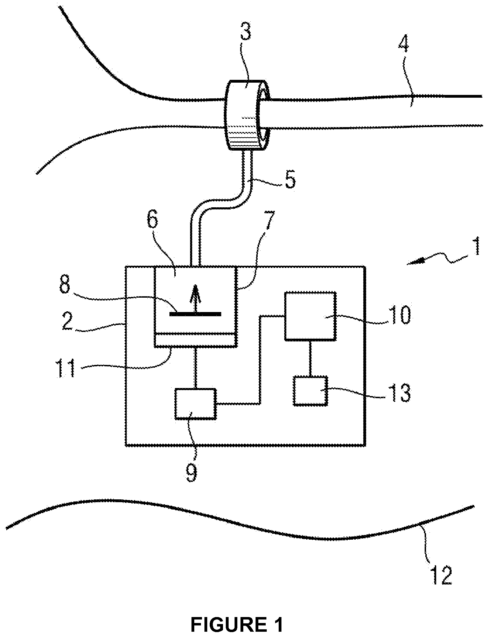

La figure 1 illustre de manière schématique le dispositif médical 1 selon l'invention selon un exemple de réalisation dans lequel il correspond à un sphincter urinaire artificiel destiné à lutter contre l'incontinence.

Le dispositif médical 1 comprend de manière générale un boîtier rigide 2 et un élément déformable 3. Dans cet exemple l'élément déformable 3 est constitué par une manchette occlusive gonflable agencée autour d'au moins une partie d'un conduit naturel 4 du corps humain ou animal, l'urètre dans le cas présent. La manchette est de préférence fabriquée en matériau élastomère biocompatible, par exemple du silicone.

Le boîtier 2 et la manchette occlusive 3 sont reliés par une connexion tubulaire 5 et sont en liaison fluidique, c'est-à-dire que le dispositif médical 1 est agencé pour déplacer du fluide en volume variable du boîtier vers la manchette et inversement. Il s'agit d'un dispositif à fonctionnement hydraulique.

Le boîtier 2 comprend un réservoir de fluide 6 à volume variable. Le réservoir 6, la connexion tubulaire 4 et la manchette occlusive 3 sont tous les trois connectés de manière fluidique de sorte à former un seul et unique circuit fluidique.

Le réservoir 6 comprend une partie fixe 7 et une partie mobile 8. La partie mobile 8, lors de son déplacement, fait varier le volume de fluide du réservoir pour le transférer du réservoir 6 vers la manchette occlusive 3, qui se gonfle alors, augmentant la pression pour occlure le conduit. Inversement, un déplacement de la partie mobile 8 dans le sens opposé entraine une augmentation du volume du réservoir, et donc un transfert de fluide de la manchette 3 vers le réservoir 6. Ceci a pour effet de relâcher la pression sur l'urètre et ainsi de permettre la miction.

La partie mobile 8 du réservoir 6 est couplée mécaniquement et entraînée par un actionneur 9 pour produire un déplacement linéaire par rapport à la partie fixe 7 de sorte à ajuster le volume du réservoir.

L'actionneur 9 peut notamment comprendre un moteur électromagnétique et un réducteur. L'actionneur est commandé par une unité de commande 10 décrite en détail ci-après et alimenté par une source d'énergie (non illustrée) rechargeable ou non. Dans une configuration particulière la source d'énergie est à l'extérieur du corps humain et transmet de l'énergie sans fil au dispositif médical 1.

L'unité 10 est appelée unité de commande mais il est entendu qu'elle réalise à la fois des fonctions de commande, des fonctions de contrôle et des fonctions de calcul, comme cela apparaîtra dans ce qui suit.

En outre, il est prévu selon l'invention, que le boîtier 2 inclut également un capteur 11 apte à mesurer ou calculer la pression dans le circuit fluidique. Le capteur 11 mesure soit directement la pression exercée, soit une force exercée et l'unité de commande 10 calcule ensuite la pression correspondante. Dans ce dernier cas, le capteur 11 est un capteur de force agencé dans le boîtier 2 et par exemple lié mécaniquement à la partie mobile 8 du réservoir 6 pour mesurer une force de traction et/ou de compression dans la direction de déplacement de la partie mobile du réservoir.

En particulier, le capteur de force est adapté pour se défléchir sous l'application d'une force de compression ou de traction. Cette déformation peut être mesurée par exemple au moyen d'une jauge de déformation ou jauge de contrainte, et la force appliquée est déterminée à partir de la déformation ainsi mesurée. En partant de la force mesurée, la surface de la partie mobile 8 étant connue, l'unité de commande 10 peut ensuite en déduire la pression correspondante, la force étant le produit de la pression par la surface.

Alternativement le capteur 11 est lié mécaniquement à l'actionneur, lui-même couplé mécaniquement avec la partie mobile 8 du réservoir 6, notamment de manière solidaire, de sorte à mesurer ou calculer la pression dans le circuit fluidique.

Selon l'invention, le boîtier 2 du dispositif médical 1 peut également inclure un capteur de position ou de posture 13 connecté à l'unité de commande 10 pour détecter la position ou la posture et/ou le changement de position ou de posture d'une partie du corps humain ou animal ou du corps entier.

Ce capteur de posture 13 se présente par exemple sous la forme d'un accéléromètre, notamment un accéléromètre agencé pour calculer les accélérations linéaires selon trois axes orthogonaux, un gyroscope et/ou un inclinomètre.

Comme illustré sur la figure 1, le réservoir 6 avec sa partie fixe 7 et sa partie mobile 8, l'actionneur 9, l'unité de commande 10, le capteur 11 et le capteur 13 sont tous agencés dans le boîtier 2 du dispositif médical 1.

De même comme montré, le dispositif médical 1 incluant le boîtier 2, la manchette 3 et la connexion tubulaire 5 sont implantés dans le corps humain ou animal qui est symbolisé par le trait 12 représentant la peau du corps sur la figure 1.

Le dispositif médical implanté 1 peut être commandé à distance par le patient au moyen d'un système de commande sans fil non-implanté dans le corps humain ou animal de type télécommande.

Dans ce cas, il est prévu que le système de commande à distance communique avec le dispositif médical via des ondes radiofréquences ou par induction électromagnétique.

Cette communication peut avoir pour but de piloter le fonctionnement du dispositif implanté, par exemple pour l'activer ou le désactiver, ouvrir ou fermer la manchette occlusive, configurer les paramètres internes du dispositif médical ou d'obtenir des informations de la part du dispositif, par exemple connaître l'état du dispositif ou obtenir des paramètres du patient mesurés par des capteurs implantés avec le dispositif.

Alternativement, il est aussi envisagé de pouvoir contrôler le dispositif médical sans avoir recours à un système de commande externe. Dans ce cas, le dispositif médical est équipé d'un autre capteur apte à détecter une action mécanique exercée sur le corps pas le patient. Cette action mécanique peut se présenter sous la forme de tapotement d'une certaine durée et fréquence représentant un code correspondant à une commande.

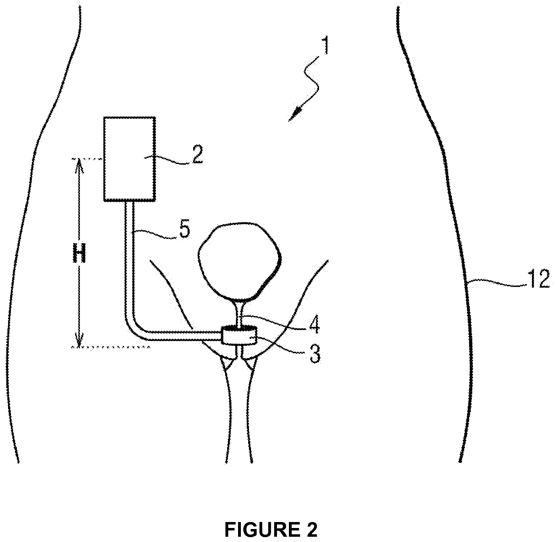

Sur la figure 2, on a illustré un corps humain 12 en position debout vue de face avec le dispositif médical 1 selon l'invention implanté.

En particulier on montre que le boîtier 2 est implanté dans l'abdomen du patient alors que la manchette occlusive 3 dans le cas du sphincter artificiel est disposée autour de l'urètre bien en dessous du boîtier. Ils sont connectés au moyen de la connexion tubulaire 5.

Le boîtier 2 et la manchette 3 sont donc implantés de manière distante l'un de l'autre dans le corps humain 12, et en particulier à une hauteur différente lorsque le patient est debout.

Dans ce qui suit, on nommera H la différence de hauteur entre le boitier 2 et la manchette en position debout, en d'autres mots cela correspond à la distance entre le boîtier 2 et la manchette 3 selon la direction verticale (direction de la gravité) lorsque le patient est en position debout. Selon l'invention, la distance H est de préférence d'au moins 10 cm, encore de préférence d'une distance d'au moins de 20 cm.

Cette différence de hauteur entraine une différence de pression entre la pression dans le boîtier 2 et la pression dans la manchette 3. Ce phénomène hydrostatique est appelé « colonne d'eau ».

En particulier, le boîtier 2 et la manchette 3 sont donc implantés de manière distante l'un de l'autre selon une direction sensiblement équivalente à une direction longitudinale dudit corps. Ce positionnement particulier induit un changement de direction de l'axe formé par le boîtier et la manchette lors d'un changement de posture du corps, notamment lors du passage d'une position debout à allongée.

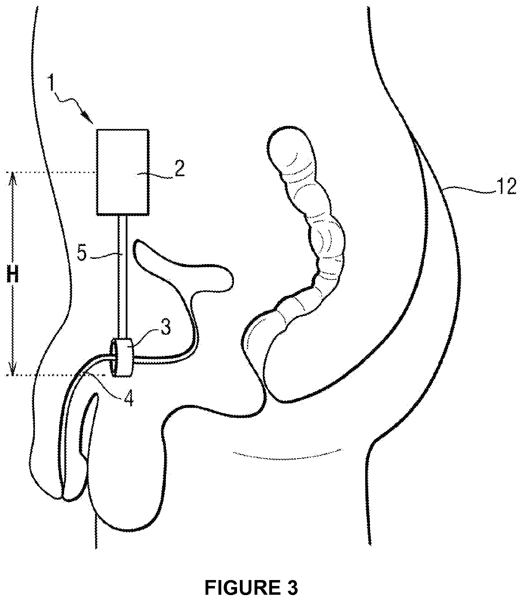

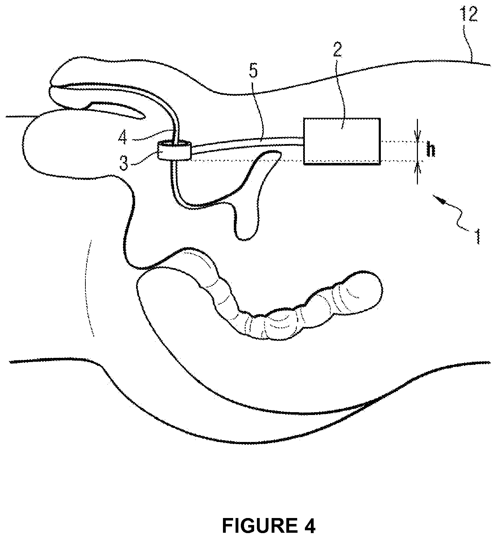

Sur les figures 3 et 4, on montre respectivement le détail du dispositif médical 1 implanté dans le corps 12 d'un homme du sexe masculin vu de profil en position debout et en décubitus dorsal.

De même que sur la figure 2, sur la figure 3, on montre le boîtier 2 et la manchette 3 implantés de manière distante l'un de l'autre dans le corps humain 12, avec une différence de hauteur H lorsque le patient est debout.

Par contre, sur la figure 4, on montre le boîtier 2 et la manchette 3 également implantés de manière distante l'un de l'autre dans le corps humain 12, mais cette fois avec une différence de hauteur h lorsque le patient est en décubitus dorsal.

On montre bien que la différence de hauteur H en position debout du patient est différente de la différence de hauteur h en décubitus dorsal. En outre, dans le cas de l'implantation particulière du dispositif médical 1 pour lutter contre l'incontinence urinaire, la différence de hauteur H en position debout est supérieure à la différence de hauteur h en décubitus dorsal.

En raison, du phénomène de « colonne d'eau » la pression mesurée par le capteur 11 est différente dans les deux positions.

En position debout, et comme le boîtier 2 est rigide et qu'il est lié de manière hermétique à l'extrémité de la connexion tubulaire connectée au réservoir, il n'y a pas de transmission directe de la pression due au phénomène de « colonne d'eau », la pression dans la manchette occlusive 3 est donc par conséquent sensiblement égale à la pression mesurée par le capteur 11 dans le réservoir 6 plus la pression correspondant à la colonne d'eau H qui se trouve au-dessus de la manchette 3 et qui, donc, applique une pression correspondante.

En décubitus dorsal (cf. figure 4), pour les mêmes raisons, la pression dans la manchette occlusive 3 est sensiblement égale à la pression mesurée par le capteur 11 dans le réservoir 6 plus la pression correspondant à la colonne d'eau h qui se trouve au-dessus de la manchette 3.

Dans le cas d'un dispositif médical 1 incluant un boîtier rigide 2 et une manchette 3 de type élément déformable, la pression dans la manchette 3 est sensiblement la même en position debout et en décubitus dorsal, alors que la pression dans le réservoir 6 mesurée par le capteur 11 est différente dans les deux positions.

Avantageusement, selon l'invention, il est donc tiré parti de cet effet, propre au dispositif médical 1, pour détecter une posture et/ou un changement de posture du corps au moyen de la mesure ou du calcul de la pression exercée dans le réservoir 6 comme cela sera expliqué plus en détail ci-après.

Il est important de noter que le sphincter urinaire artificiel en trois parties (la manchette occlusive, le ballon de régulation et la pompe) de l'état de l'art tel que décrit précédemment ne permet pas de mettre en oeuvre cette nouvelle fonction.

D'abord parce qu'il ne comprend pas de capteur apte à mesurer ou calculer la pression, puisqu'il s'agit d'un dispositif passif, mais aussi du fait de son agencement différent.

En effet, à l'inverse, dans ce dispositif de l'état l'art, le ballon de régulation étant souple et déformable, il y a une transmission directe de la pression exercée par la colonne d'eau à la manchette occlusive. Dans ce cas, c'est la pression exercée par la manchette qui varie en fonction de la position alors que la pression dans le ballon de régulation est la même en position debout et en décubitus dorsal.

Il n'est donc pas possible, comme dans l'invention, de détecter la position ou le changement de position en fonction de la pression mesurée.

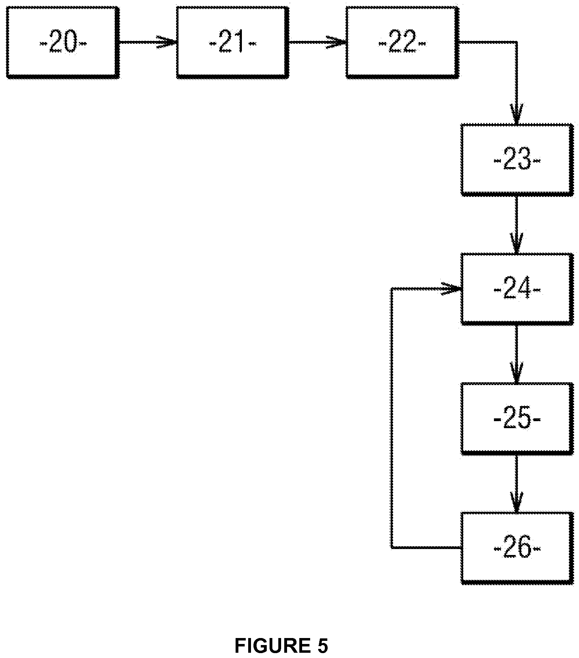

En référence à la figure 5, nous allons maintenant décrire la méthode de détection de posture ou de changement de posture selon l'invention.

La méthode de détection selon l'invention prévoit des étapes per-opératoires, des étapes d'implantation et des étapes post-opératoires.

L'étape 20 per-opératoire consiste à fournir le dispositif médical 1 comprenant le boiter 2, l'élément déformable 3 et la connexion tubulaire 5, avec à l'intérieur du boîtier 2, le réservoir 6 équipé d'une partie fixe 7 et d'une partie mobile 8, un actionneur 9, une unité de commande 10, un capteur 11 apte à mesurer ou calculer la pression dans le réservoir 6 et le capteur de posture 13 tels que décrits précédemment.

A l'étape 21, lors de l'opération, le chirurgien implante ce dispositif médical 1 afin d'obtenir la configuration illustrée sur la figure 2 avec le boîtier 2 implanté dans l'abdomen du patient, la manchette occlusive 3 distante du boîtier 3 et disposée autour de l'urètre 4.

A l'étape 22, toujours pendant l'opération, le chirurgien connecte le boîtier 2 à la manchette occlusive 3 au moyen de la connexion tubulaire 5 pour créer le circuit fluidique.

Entre les étapes 21 et 22, il est également prévu une étape de purge du dispositif (non détaillé ici) de sorte à évacuer l'air et remplir le dispositif de fluide.

Une fois implanté le dispositif médical peut être de préférence configuré en post opératoire.

Les étapes suivantes décrivent donc la configuration du dispositif médical 1 pour détecter une posture et/ou un changement de posture du corps en fonction d'une variation de la pression mesurée ou calculée par le capteur 11.

A l'étape 23, on réalise une calibration du dispositif 1. Par exemple cette étape peut consister à demander au patient de prendre des postures différentes et connues telles que passer de la position debout à décubitus dorsal. Pendant cette étape les pressions mesurées par le capteur 11 sont enregistrées dans une table dans la mémoire de l'unité de commande 10 en correspondance avec les positions, par exemple debout ou couchée, ou des positions intermédiaires.

Dans cette table de correspondance, on peut définir des seuils à partir desquels on considère que le patient est dans une position déterminée, par exemple couchée ou de debout.

Il sera expliqué ci-après comment ces mesures peuvent être combinées avec les valeurs mesurée par un capteur de posture du type accéléromètre pour rendre la détection de posture encore plus robuste et plus fiable.

L'étape de calibration 23 peut être réalisée une seule fois ou plusieurs fois dans le temps pour affiner les mesures et rendre la détection plus robuste.

A l'étape 24 de la méthode de détection, le capteur 11 mesure la pression à des intervalles réguliers de temps, de préférence fréquents pour être plus réactif quant aux détections de changement de postures ou pour détecter des changements de posture rapides. Par exemple, on mesure la pression toutes les 200 millisecondes.

A l'étape 25, on compare la valeur de pression courante à la valeur de pression précédente et aux valeurs enregistrés lors de l'étape 23 de calibration pour détecter un changement de posture et donc une nouvelle posture à l'étape 26.

Selon l'invention, l'unité de commande 10 est agencée pour répéter les étapes 24, 25 et 26 à des intervalles de temps réguliers et fréquents pour détecter les changements de postures.

Dans un mode de réalisation particulier de l'invention dans lequel le boîtier 2 du dispositif inclut un capteur de posture 13 tel que décrit précédemment, les valeurs mesurées peuvent être utilisé en complément des valeurs de pression pour la détection de posture.

Ce capteur de posture 13, par exemple un accéléromètre à trois axes, nécessite également une calibration à l'étape 23 pour permettre d'aligner virtuellement l'accéléromètre avec un repère connu. Une orientation virtuelle permet de faire correspondre les 3 axes de l'accéléromètre avec les axes liés au corps, c'est-à-dire l'axe supérieur-inférieur, l'axe postérieur-antérieur et l'axe gauche-droite. Cette orientation virtuelle est de préférence obtenue en calculant des matrices de changement de repère lorsque le patient prend des postures prédéterminées, on enregistre ensuite les accélérations correspondantes et on en déduit la matrice de changement de repère en partant du fait que la seule accélération à laquelle est soumis le corps est l'accélération gravitationnelle.

Si le dispositif médical 1 est équipé d'un tel capteur de posture 13, alors il est prévu à l'étape 24 de la méthode de détection selon l'invention, que le capteur de posture 13 mesure la posture à des intervalles réguliers de temps. De même que précédemment, il est préférable que la détection de la posture soit fréquente pour être plus réactif aux changements. Par exemple, on mesure la posture toutes les 200 millisecondes.

De même selon ce mode de réalisation, à l'étape 25, on compare la valeur correspondant à la posture. Dans l'exemple, on compare les valeurs d'accélérations courantes aux valeurs d'accélérations précédentes et aux valeurs enregistrées lors de l'étape 23 de calibration pour détecter un changement de posture et donc une nouvelle posture à l'étape 26. De préférence, à cette étape, une mesure de la pression réalisée par le capteur de pression 11, également calibré à l'étape 23, vient confirmer ou infirmer la détection de posture ou de changement de posture du capteur de posture 13. Enfin, dans ce mode, l'unité de commande 10 est également agencée pour répéter les étapes 24, 25 et 26.

De préférence, dans ce mode de réalisation particulier, c'est lorsqu'une faible variation est détectée par le capteur de posture 13 sur les mesures instantanées d'accélération, qu'une mesure de la pression est réalisée par le capteur de pression 11 permettant d'inférer la posture courante. Cette inférence est validée ou invalidée par la comparaison entre la valeur de la pression courante et la valeur de la pression liée à la position inférée lors de l'étape de calibration 23.

Le capteur de posture 13 étant généralement plus économe en énergie que le capteur de pression 11, ce mode de réalisation avec un capteur de posture 13 disponible, présente l'avantage de pouvoir économiser l'énergie globale utilisée par le dispositif médical lorsque le capteur de pression 11 n'est utilisé que pour confirmer la posture mesurée par le capteur de posture 13 et seulement dans certains cas, par exemple quand la variation mesurée par le capteur de posture est faible.

Par conséquent, la présente invention permet donc une détection de la posture ou du changement de posture plus robuste et plus fiable pour permettre d'adapter au mieux la pression exercée sur le conduit naturel par l'élément déformable et ainsi éviter de trop le solliciter et de l'endommager.

L'invention est également applicable à d'autres domaines. Elle est par exemple adaptée aux dispositifs médicaux qui appliquent une thérapie spécifique comme c'est le cas d'un stimulateur cardiaque ou d'un neurostimulateur.

Généralement, un stimulateur cardiaque fournit des impulsions électriques destinées à stimuler les muscles cardiaques permettant d'accélérer la pulsation du rythme cardiaque lorsque ce dernier est trop lent.

Dans ce cas, l'invention permettant la détection précise de la posture du corps ou un changement de cette dernière, il est possible de détecter qu'un patient est en position allongée parce qu'il vient de faire un malaise et qu'une thérapie spécifique doit être appliquée pour modifier son rythme cardiaque.

Les neurostimulateurs sont, eux, implantés dans le corps humain et connectés à la colonne vertébrale. Ils sont en outre configurés pour envoyer des signaux électriques dans l'espace épidural de manière à soulager des douleurs chroniques.

La neurostimulation agit en interrompant les signaux liés à la douleur entre la moelle épinière et le cerveau. Le cerveau ne reçoit donc plus ces signaux et le patient ne ressent plus la douleur.

Par ailleurs, certaines douleurs sont souvent amenées à s'intensifier lors d'un changement de posture, notamment lorsque le patient s'alonge, s'assoit ou se met à marcher. Avec l'invention, il devient donc possible d'adapter les différents paramètres de stimulation à ces variations de postures.

A la différence des dispositifs pour lutter contre l'incontinence urinaire, dans le cas du stimulateur cardiaque ou du neurostimulateur, l'élément déformable n'est plus agencé autour d'un conduit naturel à occlure. Mais dans ces applications, il prend la forme d'un élément déformable type ballon sans autre fonction que celle de pouvoir reproduire le phénomène de colonne d'eau. Il est donc encore disposé à l'extrémité de la connexion tubulaire et toujours implanté dans le corps de manière distante du boîtier à une hauteur différente lorsque le patient est debout. Comme précédemment décrit, il est possible d'exploiter une mesure de pression différente selon la position et donc de détecter un changement de posture. Les autres éléments du dispositif médical sont similaires et ont un fonctionnement similaire à ce qui a été décrit précédemment.

Il est entendu que les modes de réalisation décrits ci-dessus correspondent à des exemples particuliers et non-limitatifs et que diverses modifications peuvent être apportées sans pour autant s'éloigner de l'invention telle que revendiquée.

## Revendications

1. Dispositif médical (1) agencé pour être implanté dans un corps humain ou animal (12), comprenant un boîtier (2), au moins un élément déformable (3), une connexion tubulaire (5) assurant une liaison fluidique entre le boîtier (2) et l'élément déformable (3) pour former un circuit fluidique, et au moins un capteur (11) apte à mesurer ou calculer une pression dans le circuit fluidique, le boîtier (2) et l'élément déformable (3) étant adaptés pour être implantés dans le corps humain ou animal distants l'un de l'autre, le dispositif médical (1) étant **caractérisé en ce qu'**il est configuré pour détecter une position et/ou un changement de position d'au moins une partie dudit corps en fonction d'une variation de la pression mesurée ou calculée par le capteur.

2. Dispositif médical selon la revendication 1, dans lequel le boîtier (2) est rigide et lié de manière hermétique à une extrémité de ladite connexion tubulaire (5).

3. Dispositif médical selon l'une quelconque des revendications précédentes, dans lequel l'élément déformable (3) est gonflable.

4. Dispositif médical selon l'une quelconque des revendications précédentes, dans lequel le capteur (11) est disposé à l'intérieur du boîtier (2) et apte à mesurer ou calculer la pression dans l'élément déformable (3).

5. Dispositif médical selon la revendication précédente, dans lequel le boîtier (2) comprend un réservoir (6) de fluide incluant une partie mobile (8) et un actionneur (9) de la partie mobile pour faire varier un volume dudit réservoir, le capteur (11) apte à mesurer ou calculer la pression étant lié mécaniquement à ladite partie mobile (8) et/ou à l'actionneur (9), notamment de manière solidaire.

6. Dispositif médical selon l'une quelconque des revendications précédentes, dans lequel le boîtier (2) et l'élément déformable (3) sont adaptés pour être implantés dans le corps humain ou animal distants l'un de l'autre selon une direction qui varie lors d'un changement de position d'au moins une partie dudit corps, notamment lors du passage d'une position debout à allongée.

7. Dispositif médical selon la revendication précédente, dans lequel le boîtier (2) et l'élément déformable (3) sont adaptés pour être implantés dans le corps humain ou animal distants l'un de l'autre selon une direction sensiblement équivalente à une direction longitudinale dudit corps.

8. Dispositif médical selon l'une quelconque des revendications précédentes, dans lequel le boîtier (2) et l'élément déformable (3) sont adaptés pour être implantés dans le corps humain ou animal distants l'un de l'autre d'une distance d'au moins 5 cm, de préférence d'une distance d'au moins 10 cm, encore de préférence d'une distance d'au moins de 20 cm.

9. Dispositif médical selon l'une quelconque des revendications précédentes, dans lequel le dispositif (1) comprend un capteur de position (13) d'au moins une partie dudit corps humain ou animal.

10. Dispositif médical selon l'une quelconque des revendications 1 à 8, dans lequel le dispositif comprend un capteur de position (13) d'au moins une partie du corps humain ou animal, le dispositif étant configuré pour détecter la position et/ou le changement de position d'au moins une partie dudit corps à partir d'une mesure ou d'un calcul de pression par le capteur (11) apte à mesurer ou calculer la pression dans le circuit fluidique et pour confirmer ladite position et/ou ledit changement de position à partir d'une mesure du capteur de position (13).

11. Dispositif médical selon l'une quelconque des revendications 1 à 8, dans lequel le dispositif comprend un capteur de position (13) d'au moins une partie du corps humain ou animal, le dispositif étant configuré pour détecter la position et/ou le changement de position d'au moins une partie dudit corps par le capteur de position (13) et pour confirmer ladite position et/ou ledit changement de position à partir d'une mesure ou d'un calcul de la pression dans le circuit fluidique par le capteur (11) apte à mesurer ou calculer la pression.

12. Dispositif médical selon l'une quelconque des revendications 9 à 11, dans lequel le capteur de position (13) est choisi parmi : un accéléromètre, notamment un accéléromètre agencé pour calculer les accélérations linéaires selon trois axes orthogonaux, un gyroscope et/ou un inclinomètre.

13. Dispositif médical selon l'une quelconque des revendications précédentes, dans lequel le dispositif comprend une unité de commande (10) configurée pour réaliser au moins une des opérations suivantes :
- mesurer ou calculer une pression au moyen du capteur apte à mesurer ou calculer une pression,
- détecter une position ou un changement de position d'au moins une partie dudit corps en fonction d'un capteur de position d'au moins une partie dudit corps humain ou animal et/ou du capteur apte à mesurer ou calculer la pression.

14. Dispositif médical selon la revendication 13 en combinaison avec la revendication 5, dans lequel l'unité de commande (10) est configurée pour commander l'actionneur pour déplacer la partie mobile du réservoir et faire varier le volume dudit réservoir.

15. Dispositif médical selon l'une quelconque des revendications précédentes, dans lequel l'élément déformable (3) est une manchette occlusive agencée pour obturer un conduit naturel (4) dudit corps humain ou animal.

16. Dispositif médical selon l'une quelconque des revendications 1 à 15, dans lequel le dispositif est configuré pour être implanté dans un corps humain ou animal pour obturer un conduit naturel (4) dudit corps humain ou animal compris parmi au moins : un urètre, un conduit gastrique, un colon ou un rectum.

17. Dispositif médical selon l'une quelconque des revendications 1 à 14, dans lequel le dispositif est configuré pour agir en tant que stimulateur cardiaque ou tant que neurostimulateur.

18. Méthode de détection d'une position et/ou d'un changement de position d'au moins une partie d'un corps humain ou animal, dans laquelle ledit corps comprend un dispositif médical (1) comprenant un boîtier (2), au moins un élément déformable (3), une connexion tubulaire (11) assurant une liaison fluidique entre le boîtier (2) et l'élément déformable (3) pour former un circuit fluidique, et au moins un capteur (11) apte à mesurer ou calculer une pression dans le circuit fluidique, le boîtier (2) et l'élément déformable (3) étant agencés à distance l'un de l'autre, dans laquelle on détecte une position et/ou un changement de position d'au moins une partie dudit corps en fonction d'une variation de la pression mesurée ou calculée par le capteur (11) apte à mesurer ou calculer une pression.

19. Méthode de détection selon la revendication précédente, dans laquelle le boîtier (2) et l'élément déformable (3 ; 31 ; 41) sont distants l'un de l'autre selon une direction qui varie lors d'un changement de position d'au moins une partie dudit corps, notamment lors du passage d'une position debout à allongée.

20. Méthode de détection selon la revendication précédente, dans laquelle le boîtier (2) et l'élément déformable (3 ; 31 ; 41) sont distants l'un de l'autre selon une direction sensiblement équivalente à une direction longitudinale dudit corps.

21. Méthode de détection selon l'une quelconque des revendications 18 à 20, dans laquelle le dispositif médical comprend une unité de commande (10) qui réalise au moins une des opérations suivantes :
- mesurer ou calculer une pression au moyen du capteur (11) apte à mesurer ou calculer une pression,
- détecter une position et/ou un changement de position d'au moins une partie dudit corps en fonction d'un capteur de position (13) et/ou du capteur (11) apte à mesurer ou calculer la pression.

22. Méthode de détection selon la revendication 21, dans laquelle l'unité de commande (10) est configurée pour commander un actionneur (9) pour déplacer une partie mobile (8) d'un réservoir (6) et faire varier le volume dudit réservoir.

23. Méthode de détection selon l'une quelconque des revendications 18 à 22, dans laquelle on calibre un capteur de position (13) apte à détecter une position et/ou un changement de position d'au moins une partie du corps au moyen du capteur (11) apte à mesurer ou calculer une pression.

24. Méthode de détection selon l'une quelconque des revendications 18 à 23, dans laquelle l'élément déformable (3) est gonflable et choisi parmi une manchette occlusive agencée pour obturer un conduit naturel dudit corps humain ou animal.

## Patentansprüche

1. Medizinische Vorrichtung (1), die für eine Implantation in einen Körper eines Menschen oder eines Tieres (12) eingerichtet ist, umfassend ein Gehäuse (2), mindestens ein verformbares Element (3), eine Schlauchverbindung (5), die eine Fluidverbindung zwischen dem Gehäuse (2) und dem verformbaren Element (3) sichert, um einen Fluidkreis zu bilden, und mindestens einen Sensor (11), der imstande ist, einen Druck in dem Fluidkreis zu messen oder zu berechnen, wobei das Gehäuse (2) und das verformbare Element (3) dafür geeignet sind, voneinander beabstandet in den Körper eines Menschen oder eines Tieres implantiert zu werden, wobei die medizinische Vorrichtung (1) **dadurch gekennzeichnet ist, dass** sie ausgelegt ist, um eine Position und/oder einen Positionswechsel mindestens eines Teils des Körpers in Abhängigkeit von einer Veränderung des von dem Sensor gemessenen oder berechneten Drucks zu ermitteln.

2. Medizinische Vorrichtung nach Anspruch 1, wobei das Gehäuse (2) starr ist und mit einem Ende der Schlauchverbindung (5) hermetisch verbunden ist.

3. Medizinische Vorrichtung nach einem der vorangehenden Ansprüche, wobei das verformbare Element (3) aufblasbar ist.

4. Medizinische Vorrichtung nach einem der vorangehenden Ansprüche, wobei der Sensor (11) im Inneren des Gehäuses (2) angeordnet und geeignet ist, den Druck in dem verformbaren Element (3) zu messen oder zu berechnen.

5. Medizinische Vorrichtung nach dem vorangehenden Anspruch, wobei das Gehäuse (2) einen Fluidbehälter (6) umfasst, der einen beweglichen Teil (8) und einen Aktuator (9) des beweglichen Teils einschließt, um ein Volumen des Behälters zu verändern, wobei der Sensor (11), der geeigntet ist, den Druck zu messen oder zu berechnen, mit dem beweglichen Teil (8) und/oder mit dem Aktuator (9), mechanisch verbunden, insbesondere starr verbunden, ist.

6. Medizinische Vorrichtung nach einem der vorangehenden Ansprüche, wobei das Gehäuse (2) und das verformbare Element (3) geeignet sind, in den Körper eines Menschen oder eines Tieres beabstandet voneinander implantiert zu werden entlang einer Richtung, die sich bei einem Positionswechsel von mindestens einem Teil des Körpers ändert, insbesondere bei einem Wechseln aus einer aufrechten Position in eine liegende Position.

7. Medizinische Vorrichtung nach vorangehendem Anspruch, wobei das Gehäuse (2) und das verformbare Element (3) geeignet sind in den Körper eines Menschen oder eines Tieres beabstandet voneinander implantiert zu werden entlang einer Richtung, die näherungsweise äquivalent zu einer Längsrichtung des Körpers ist.

8. Medizinische Vorrichtung nach einem der vorangehenden Ansprüche, wobei das Gehäuse (2) und das verformbare Element (3) geeignet sind, in den Körper eines Menschen oder eines Tieres beabstandet voneinander in einem Abstand von mindestens 5 cm, bevorzugterweise in einem Abstand von mindestens 10 cm, am meisten bevorzugt in einem Abstand von mindestens 20 cm implantiert zu werden.

9. Medizinische Vorrichtung nach einem der vorangehenden Ansprüche, wobei die Vorrichtung (1) einen Positionssensor (13) mindestens eines Teils des Körpers eines Menschen oder eines Tieres umfasst.

10. Medizinische Vorrichtung nach einem der Ansprüche 1 bis 8, wobei die Vorrichtung einen Positionssensor (13) mindestens eines Teils des Körpers eines Menschen oder eines Tieres umfasst, wobei die Vorrichtung dazu ausgelegt ist, die Position und/oder den Positionswechsel mindestens eines Teils des Körpers ausgehend von einer Messung oder einer Berechnung eines Drucks durch den Sensor (11), der imstande ist, den Druck im Fluidkreis zu messen oder zu berechnen, zu ermitteln und die Position und/oder den Positionswechsel ausgehend von einer Messung des Positionssensors (13) zu bestätigen.

11. Medizinische Vorrichtung nach einem der Ansprüche 1 bis 8, wobei die Vorrichtung einen Positionssensor (13) mindestens eines Teils des Körpers eines Menschen oder eines Tieres umfasst, wobei die Vorrichtung dazu ausgelegt ist, die Position und/oder den Positionswechsel mindestens eines Teils des Körpers durch den Positionssensor (13) zu ermitteln und die Position und/oder den Positionswechsel ausgehend von einer Messung oder einer Berechnung des Drucks im Fluidkreis durch den Sensor (11), der geeignet ist, den Druck zu messen oder zu berechnen, zu bestätigen.

12. Medizinische Vorrichtung nach einem der Ansprüche 9 bis 11, wobei der Positionssensor (13) ausgewählt ist aus: einem Beschleunigungsmesser, insbesondere einem Beschleunigungsmesser, der eingerichtet ist, um die linearen Beschleunigungen in drei orthogonalen Achsen zu berechnen, einem Gyroskop und/oder einem Neigungsmesser.

13. Medizinische Vorrichtung nach einem der vorangehenden Ansprüche, wobei die Vorrichtung eine Steuereinheit (10) umfasst, die dazu ausgelegt ist, mindestens eine der folgenden Operationen durchzuführen:
- Messen oder Berechnen eines Drucks mittels des Sensors, der imstande ist, einen Druck zu messen oder zu berechnen,
- Ermitteln einer Position oder eines Positionswechsel mindestens eines Teils des Körpers in Abhängigkeit von einem Positionssensor mindestens eines Teils des Körpers eines Menschen oder eines Tieres und/oder dem Sensor, der imstande ist, den Druck zu messen oder zu berechnen.

14. Medizinische Vorrichtung nach Anspruch 13 in Kombination mit Anspruch 5, wobei die Steuereinheit (10) dazu ausgelegt ist, den Aktuator zu steuern, um den beweglichen Teil des Behälters zu verlagern und das Volumen des Behälters zu verändern.

15. Medizinische Vorrichtung nach einem der vorangehenden Ansprüche, wobei das verformbare Element (3) eine Verschlussmanschette ist, die eingerichtet ist, um einen natürlichen Leitungskanal (4) des Körpers eines Menschen oder eines Tieres zu verschließen.

16. Medizinische Vorrichtung nach einem der Ansprüche 1 bis 15, wobei die Vorrichtung dazu ausgelegt ist, in den Körper eines Menschen oder eines Tieres implantiert zu sein, um einen natürlichen Leitungskanal (4) des Körpers eines Menschen oder eines Tiers zu verschließen, zu dem mindestens gehört: ein Harnleiter, ein Magenkanal, ein Kolon oder ein Rektum.

17. Medizinische Vorrichtung nach einem der Ansprüche 1 bis 14, wobei die Vorrichtung dazu ausgelegt ist, als Herzstimulator oder als Neurostimulator zu wirken.

18. Methode zur Ermittlung einer Position und/oder eines Positionswechsels mindestens eines Teils eines Körpers eines Menschen oder eines Tieres, wobei der Körper eine medizinische Vorrichtung (1) umfasst, die ein Gehäuse (2), mindestens ein verformbares Element (3), eine Schlauchverbindung (11), die eine Fluidverbindung zwischen dem Gehäuse (2) und dem verformbaren Element (3) sichert um einen Fluidkreis zu bilden, und mindestens einen Sensor (11), der imstande ist, einen Druck in dem Fluidkreis zu messen oder zu berechnen, umfasst, wobei das Gehäuse (2) und das verformbare Element (3) voneinander beabstandet angeordnet sind, wobei eine Position und/oder ein Positionswechsel mindestens eines Teils des Körpers in Abhängigkeit von einer Schwankung des von dem Sensor (11), der geeignet ist, einen Druck zu messen oder zu berechnen, gemessenen oder berechneten Druck ermittelt wird.

19. Ermittlungsmethode nach vorangehendem Anspruch, wobei das Gehäuse (2) und das verformbare Element (3; 31; 41) in einer Richtung voneinander beabstandet sind, die sich bei einem Positionswechsel mindestens eines Teils des Körpers, insbesondere bei einem Wechsel aus einer aufrechten Position in eine liegende, ändert.

20. Ermittlungsmethode nach vorangehendem Anspruch, wobei das Gehäuse (2) und das verformbare Element (3; 31; 41) in einer Richtung voneinander beabstandet sind, die näherungsweise einer Längsrichtung des Körpers äquivalent ist.

21. Ermittlungsmethode nach einem der Ansprüche 18 bis 20, wobei die medizinische Vorrichtung eine Steuereinheit (10) umfasst, die mindestens eine der folgenden Operationen durchführt:
- Messen oder Berechnen eines Drucks mittels des Sensors (11), der geeignet ist, einen Druck zu messen oder zu berechnen,
- Ermitteln einer Position und/oder eines Positionswechsel mindestens eines Teils des Körpers in Abhängigkeit von einem Positionssensor (13) und/oder dem Sensor (11), der geeignet ist, den Druck zu messen oder zu berechnen.

22. Ermittlungsmethode nach Anspruch 21, wobei die Steuereinheit (10) dazu ausgelegt ist, einen Aktuator (9) zu steuern, um einen beweglichen Teil (8) eines Behälters (6) zu verlagern und das Volumen des Behälters zu verändern.

23. Ermittlungsmethode nach einem der Ansprüche 18 bis 22, wobei ein Positionssensor (13) kalibriert wird, der geeignet ist, eine Position und/oder einen Positionswechsel mindestens eines Teils des Körpers mittels des Sensors (11) zu ermitteln, der geeignet ist, einen Druck zu messen oder zu berechnen.

24. Ermittlungsmethode nach einem der Ansprüche 18 bis 23, wobei das verformbare Element (3) aufblasbar ist und aus einer Verschlussmanschette ausgewählt ist, die eingerichtet ist, um einen natürlichen Leitungskanal des Körpers eines Menschen oder eines Tieres zu verschließen.

## Claims

1. Medical device (1) arranged to be implanted in a human or animal body (12), comprising a housing (2), at least one deformable element (3), a tubular connection (5) providing a fluidic link between the housing (2) and the deformable element (3) to form a fluidic circuit, and at least one sensor (11) capable of measuring or calculating a pressure in the fluidic circuit, the housing (2) and the deformable element (3) being adapted to be implanted in the human or animal body at a distance from each other, the medical device (1) being **characterised in that** it is configured to detect a position and/or a change in position of at least one part of the said body as a function of a variation in the pressure measured or calculated by the sensor.

2. A medical device as claimed in claim 1, wherein the housing (2) is rigid and hermetically bonded to one end of said tubular connection (5).

3. A medical device according to any one of the preceding claims, wherein the deformable element (3) is inflatable.

4. A medical device according to any one of the preceding claims, wherein the sensor (11) is disposed inside the housing (2) and is capable of measuring or calculating the pressure in the deformable element (3).

5. Medical device according to the preceding claim, wherein the housing (2) comprises a fluid reservoir (6) including a movable part (8) and an actuator (9) of the movable part for varying a volume of said reservoir, the sensor (11) capable of measuring or calculating the pressure being mechanically linked to said movable part (8) and/or to the actuator (9), in particular in an integral manner.

6. A medical device according to any one of the preceding claims, wherein the housing (2) and the deformable element (3) are adapted to be implanted in the human or animal body at a distance from each other in a direction which varies when there is a change in position of at least part of the said body, in particular when passing from a standing to a lying position.

7. A medical device according to the preceding claim, wherein the housing (2) and the deformable element (3) are adapted to be implanted in the human or animal body at a distance from each other in a direction substantially equivalent to a longitudinal direction of said body.

8. A medical device according to any one of the preceding claims, wherein the housing (2) and the deformable element (3) are adapted to be implanted in the human or animal body at a distance of at least 5 cm, preferably at least 10 cm, more preferably at a distance of at least 20 cm.

9. A medical device according to any one of the preceding claims, wherein the device (1) comprises a position sensor (13) for at least one part of said human or animal body.

10. A medical device according to any one of claims 1 to 8, wherein the device comprises a position sensor (13) of at least one part of the human or animal body, the device being configured to detect the position and/or change in position of at least one part of said body from a measurement or calculation of pressure by the sensor (11) capable of measuring or calculating pressure in the fluid circuit and to confirm said position and/or change in position from a measurement of the position sensor (13).

11. A medical device according to any one of claims 1 to 8, wherein the device comprises a position sensor (13) of at least one part of the human or animal body, the device being configured to detect the position and/or change in position of at least one part of said body by the position sensor (13) and to confirm said position and/or change in position from a measurement or calculation of the pressure in the fluid circuit by the sensor (11) capable of measuring or calculating the pressure.

12. A medical device according to any one of claims 9 to 11, wherein the position sensor (13) is selected from: an accelerometer, in particular an accelerometer arranged to calculate linear accelerations along three orthogonal axes, a gyroscope and/or an inclinometer.

13. A medical device according to any one of the preceding claims, wherein the device comprises a control unit (10) configured to perform at least one of the following operations:
- measure or calculate a pressure by means of the sensor capable of measuring or calculating a pressure,
- detecting a position or a change in position of at least one part of the said body as a function of a sensor of the position of at least one part of the said human or animal body and/or of the sensor capable of measuring or calculating pressure.

14. The medical device of claim 13 in combination with claim 5, wherein the control unit (10) is configured to control the actuator to move the movable part of the reservoir and vary the volume of said reservoir.

15. A medical device according to any one of the preceding claims, wherein the deformable element (3) is an occlusive cuff arranged to close off a natural duct (4) of said human or animal body.

16. A medical device according to any one of claims 1 to 15, wherein the device is configured to be implanted in a human or animal body in order to obturate a natural duct (4) of said human or animal body including at least: a urethra, a gastric duct, a colon or a rectum.

17. A medical device according to any one of claims 1 to 14, wherein the device is configured to act as a pacemaker or as a neurostimulator.

18. A method for detecting a position and/or a change in position of at least one part of a human or animal body, wherein said body comprises a medical device (1) comprising a housing (2), at least one deformable element (3), a tubular connection (11) providing a fluid connection between the housing (2) and the deformable element (3) to form a fluid circuit, and at least one sensor (11) capable of measuring or calculating a pressure in the fluid circuit, the housing (2) and the deformable element (3) being arranged at a distance from each other, wherein a position and/or a change in position of at least one part of the said body is detected as a function of a variation in the pressure measured or calculated by the sensor (11) capable of measuring or calculating a pressure.

19. Detection method according to the preceding claim, wherein the housing (2) and the deformable element (3; 31; 41) are distant from each other in a direction which varies when there is a change in position of at least part of the said body, in particular when passing from a standing position to a lying position.

20. A detection method according to the preceding claim, wherein the housing (2) and the deformable element (3; 31; 41) are spaced apart in a direction substantially equivalent to a longitudinal direction of said body.

21. A detection method according to any one of claims 18 to 20, wherein the medical device comprises a control unit (10) which performs at least one of the following operations:
- measuring or calculating a pressure by means of the sensor (11) capable of measuring or calculating a pressure,
- detecting a position and/or a change in position of at least part of said body as a function of a position sensor (13) and/or the sensor (11) capable of measuring or calculating pressure.

22. A detection method as claimed in claim 21, wherein the control unit (10) is configured to control an actuator (9) to move a movable part (8) of a fluid reservoir (6) and vary the volume of said fluid reservoir.

23. Detection method according to any one of claims 18 to 22, wherein a position sensor (13) capable of detecting a position and/or a change in position of at least one part of the body is calibrated by means of the sensor (11) capable of measuring or calculating a pressure.

24. Detection method according to any one of claims 18 to 23, wherein the deformable element (3) is inflatable and chosen from an occlusive cuff arranged to close off a natural duct of the said human or animal body.
